# EUROPEAN PATENT APPLICATION

(11) **EP 4 357 099 A1**
(43) Date of publication of application: **24.04.2024**
(21) Application number: 21945667.0
(22) Date of filing: 29.07.2021
(51) Int. Cl.: B29C 39/26, B29C 33/44

(54) **MANUFACTURING DEVICE AND MANUFACTURING METHOD FOR MOLDS**

(30) Priority: 18.06.2021 CN 202110678575
(71) Applicant: Suzhou Reveda Medical Biotech Co., Ltd., Suzhou, Jiangsu 215200 (CN)
(72) Inventor: HUANG, Yuan, Suzhou, Jiangsu 215200 (CN); YAN, Ping, Suzhou, Jiangsu 215200 (CN); LIU, Long, Suzhou, Jiangsu 215200 (CN); CHEN, Binwen, Suzhou, Jiangsu 215200 (CN); QU, Qiuyu, Suzhou, Jiangsu 215200 (CN)
(74) Representative: Patentship Patentanwaltsgesellschaft mbH
(86) International application number: PCT/CN2021/109125
(87) International publication number: WO 2022/262086

(57) **Abstract**

The present invention provides a manufacturing device and a manufacturing method for molds. The manufacturing device includes a molding assembly including a mold table and a male mold. The mold table has an upper surface defining a depression thereon, and the male mold is configured to be disposed on a bottom surface of the depression. A height of the male mold is smaller than a depth of the depression. This manufacturing device can utilize a transfer molding method to manufacture molds in batches with higher mold production efficiency.

## Description

### TECHNICAL FIELD

The present invention relates the technical field of mold manufacturing and, in particular, to a manufacturing device and a manufacturing method for molds.

### BACKGROUND

Fabrication of polymer microneedles requires the use of silicone female molds. Such molds can be manufactured using various methods, one of which is to create microneedle-forming cavities in a solid silicone plate by burning away unwanted portions of the plate using a high-energy laser beam. Another method is transfer molding.

The laser beam approach is simple, fast and is suitable for manufacturing large-area silicone female molds. However, molds fabricated using this method tend to have rough surface morphology and hard-to-remove residues remaining in the resulting microneedle-forming cavities. All these are detrimental to the subsequent use of the manufactured molds.

The transfer molding method is also associated with a considerable number of drawbacks. A large-area silicone female mold manufactured using this method readily tends to have a substrate with an uneven thickness. When this mold is subsequently used to fabricate microneedles, the polymer solution tends to sink over the thinner portions, leading to low yield. Moreover, this method is time-consuming because it involves a sequence of processes including solution preparation, casting, degassing, heating, cooling and demolding. Once the silicone solution is prepared, its viscosity will continually rise due to the ambient temperature, storage time and other factors. This will eventually degrade the quality of the resulting silicone female molds. In addition, as the overall manufacturing process is susceptible to many factors, there is a lack of consistency among silicone female molds manufactured in different batches, making the method unsuitable for use in mass production. Another implementation of transfer molding utilizes injection molding equipment to perform an injection molding procedure on a male mold for microneedle fabrication. However, this requires the use of a mold base compatible with the male mold, which raises production cost, reduces flexibility in the manufacture of silicone female molds of various specifications and prolongs the time required for manufacture. Further, since a very high pressure is involved in the injection molding process, the male mold must be sufficiently strong, limiting the types of materials suitable for this purpose and resulting in additional increases in production cost.

### SUMMARY OF THE INVENTION

It is an object of the present invention to provide a manufacturing device and a manufacturing method for molds, which are capable of manufacturing molds in batches.

To this end, the present invention provides a manufacturing device for molds comprising a molding assembly comprising a mold table and a male mold. The mold table has an upper surface defining a depression thereon, and the male mold is configured to be disposed on a bottom surface of the depression. The male mold has a height smaller than a depth of the depression.

Optionally, the male mold may comprise a substrate having a flat upper surface, wherein the manufacturing device further comprises an adjustment mechanism for driving the male mold to move and making the upper surface of the substrate horizontal.

Optionally, both a lower surface of the substrate and the bottom surface of the depression may be flat, wherein the adjustment mechanism is configured to drive the mold table to move so that the bottom surface of the depression is located in a horizontal plane, and accordingly drive the male mold to move so that the upper surface of the substrate is located in another horizontal plane.

Optionally, the adjustment mechanism may comprise a motion table configured to move under an action of an external force, the motion table disposed under the molding assembly so as to support the molding assembly thereon.

Optionally, the depression may be configured for loading a liquid raw material therein, wherein a sealing ring for preventing flow of the liquid raw material into a space between the lower surface of the substrate and the bottom surface of the depression is disposed on the lower surface of the substrate and/or the bottom surface of the depression.

Optionally, the depression may be configured for loading a liquid raw material therein so that the male mold is submerged in the liquid raw material which is to be solidified to form a mold, and wherein the manufacturing device further comprises a demolding and removal mechanism for separating the formed mold from the molding assembly.

Optionally, the demolding and removal mechanism may comprise a demolding assembly and a mold removal assembly, the demolding assembly comprising a first telescopic rod and a first drive device, the first telescopic rod disposed vertically, the first telescopic rod passed through the mold table and configured to the male mold, the first drive device connected to drive the first telescopic rod to extend or retract, the mold removal assembly comprising a second telescopic rod, a second drive device and a support member, the second telescopic rod disposed horizontally, the second drive device configured to drive the second telescopic rod to extend or retract, the support member connected to the second telescopic rod and configured to support the mold,
wherein the manufacturing device is configured so that, when the first drive device drives the first telescopic rod to extend, the first telescopic rod propels the male mold to move upward to separate the male mold and the formed mold thereon from the mold table, that when the second drive device drives the second telescopic rod to extend, the second telescopic rod propels the support member to move so that the formed mold is supported on the support member, and that when the first drive device drives the first telescopic rod to retract, the male mold is moved downward to separate the male mold from the formed mold.

Optionally, the molding apparatus may be further configured so that, when the second drive device drives the second telescopic rod to retract, the second telescopic rod propels the formed mold to move away from the molding assembly.

Optionally, the male mold may be detachably connected to the first telescopic rod.

Optionally, the manufacturing device may further comprise a box and a vacuum generation element, the box defining thereon a mold removal port provided thereat with a door assembly, the door assembly configured to close the mold removal port, the vacuum generation element configured to create a vacuum in the box,
wherein both the molding assembly and the demolding assembly are at least partially disposed in the box; the mold removal assembly is disposed outside the box; and the second telescopic rod is configured to extend through the mold removal port so that the support member enters into the box to allow the formed mold to be supported thereon.

Optionally, the manufacturing device may further comprise a heater assembly for heating the molding assembly.

Optionally, the manufacturing device may further comprise a feeder assembly for casting a liquid raw material into the depression.

Optionally, the liquid raw material may comprise at least two components, wherein the feeder assembly comprises a plurality of reservoirs and plurality of metering mechanisms, each of the reservoirs configured to store one of the components of the liquid raw material, the metering mechanisms disposed in correspondence with the respective reservoirs and configured to measure amounts of the respective components of the liquid raw material; and
the feeder assembly further comprises a mixing mechanism for homogenously mixing the components of the liquid raw material and casting the mixed liquid raw material into the depression.

Optionally, the manufacturing device may further comprise a control assembly communicatively connected to the feeder assembly and configured to control at least one of the amounts of the components of the liquid raw material, a mixing speed of the components and a casting speed of the liquid raw material. Alternatively or additionally, when the manufacturing device comprises the box and the vacuum generation element, the control assembly may be further connected to the vacuum generation element and configured to control the vacuum in the box. Alternatively or additionally, the control assembly may be further communicatively connected to the heater assembly and configured to control a temperature of the molding assembly within a predetermined range.

Optionally, the male mold may comprise a substrate and needles arranged on an upper surface of the substrate.

To the above end, the present invention also provides a manufacturing method for molds, based on the manufacturing device for molds as defined above, and the method comprises:
Step S 10: casting a liquid raw material into the depression so that the male mold is submerged in the liquid raw material; and
Step S20: solidifying the liquid raw material to form a mold.

Optionally, the method may further comprise, prior to Step S10, Step S00: driving the male mold to move so that an upper surface of a substrate in the male mold is located in a horizontal plane.

Optionally, the liquid raw material may comprise at least two components, and the manufacturing device further comprising the feeder assembly,
wherein Step S10 comprises the feeder assembly mixing the components at a mixture ratio of the liquid raw material and casting the mixed liquid raw material into the depression at a predetermined speed.

Optionally, Step S20 may comprise heating the molding assembly to a first predetermined temperature to solidify the liquid raw material.

Optionally, the method may further comprise Step S01: heating the molding assembly to a second predetermined temperature,
wherein Step S01 is performed after Step S00 and before Step S10.

Optionally, the molding assembly may be housed in a box,
wherein the method further comprises Step S02: creating a vacuum in the box; Step S02 is performed before Step S10;
the method further comprises Step S11: introducing air into the box to restore a pressure in the box to an ambient atmospheric pressure; and Step S11 is performed after Step S10.

Compared with the prior art, the manufacturing device and manufacturing method for molds according to the present invention have the advantages as follows:
The manufacturing device for molds includes a molding assembly including a mold table and a male mold. The mold table has an upper surface defining a depression thereon, and the male mold is configured to be disposed on a bottom surface of the depression. The male mold has a height smaller than a depth of the depression. As such, a liquid raw material can be cast into the depression so that the male mold is submerged therein, and a mold matching the geometry of the male mold will form after the liquid raw material solidifies. This manufacturing device can be used to manufacture such molds in batches with higher production efficiency.

Moreover, the male mold may include a substrate with a flat upper surface, and the manufacturing device may further include an adjustment mechanism for driving the male mold to move and thereby making the upper surface of the substrate horizontal. The portion of the liquid raw material between its upper surface and the upper surface of the substrate, when solidifying, will form a substrate of the mold. Thus, placing the upper surface of the male mold in a horizontal plane by manipulating the adjustment mechanism before the liquid raw material is cast means that the upper surface of the cast liquid raw material will be parallel to the upper surface of the male mold. This allows the substrate of the mold to have a uniform thickness.

In addition, the manufacturing device may further include a demolding and removal mechanism, which can remove the mold and transfer it away immediately after the liquid raw material solidifies. Therefore, when the manufacturing device is used for continuously manufacturing molds, cooling of molds can be dispensed with, leading to an increase in production efficiency.

Further, the manufacturing device may further include a feeder assembly optionally including a plurality of reservoir, a plurality of metering mechanism and a mixing mechanism. The reservoirs may store respective components of the liquid raw material, and the metering mechanisms may determine amounts of the respective components according to an intended mixture ratio of the components. The mixing mechanism may then mix the components and casting the resulting liquid raw material into the depression. This allows in-situ preparation of the liquid raw material immediately before its use, avoiding the quality of the resulting mold from being adversely affected due to long-time storage of the liquid raw material after it is prepared.

Furthermore, the manufacturing device may further include a control assembly configured to control various parameters of the molding process. For example, the control assembly may be communicatively connected to the feeder assembly, in order to control supply of the liquid raw material. It may also be communicatively connected to the heater assembly, in order to control, for example, a heating temperature and thereby improve consistency of the manufactured molds in different batches.

### BRIEF DESCRIPTION OF THE DRAWINGS

The accompanying drawings are provided to facilitate a better understanding of the present invention and do not unduly limit the scope thereof in any sense, in which:
Fig. 1 is a schematic diagram showing the structure of a manufacturing device for molds according to an embodiment of the present invention; and
Fig. 2 is a schematic enlarged view of part of a manufacturing device for molds according to an embodiment of the present invention.

List of Reference Numerals in Drawings
100 - Molding Assembly; 110 - Mold Table; 111 - Depression; 120 - Male Mold; 121 - Substrate; 122 - Needles;
200 - Adjustment Mechanism;
310 - Electric Heating Plate;
410 - Box; 411 - Door Assembly; 420 - Vacuum Generation Element;
500 - Feeder Assembly; 510 - Reservoir; 520 - Metering Mechanism; 530 - Mixing Mechanism;
600 - Demolding and Removal Mechanism; 610 - Demolding Assembly; 611 - First Drive Device; 612 - First Telescopic Rod; 620 - Mold Removal Assembly;
621 - Second Drive Device; 622 - Second Telescopic Rod; 623 - Support Member;
700 - Control Assembly; 710 - First Control Element; 720 - Second Control Element.

### DETAILED DESCRIPTION

Particular embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. Throughout these drawings, like numerals indicate like elements.

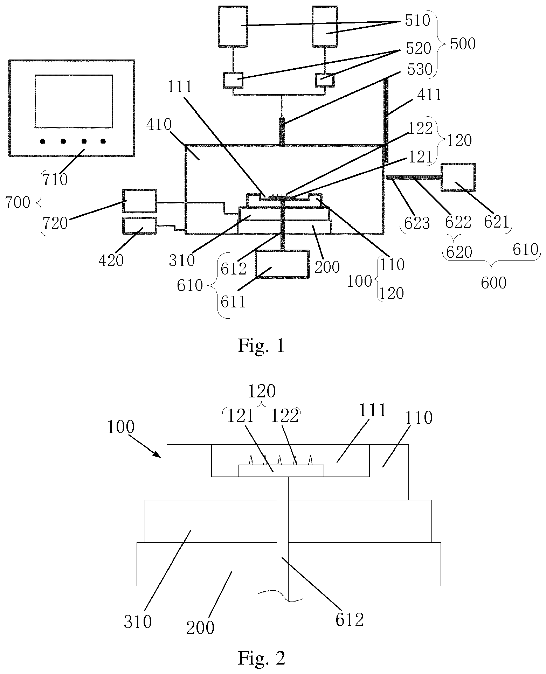

Fig. 1 is a schematic diagram showing the structure of a manufacturing device for molds according to an embodiment of the present invention, and Fig. 2 is schematic enlarged view of part of the manufacturing device. Referring to Figs. 1 and 2, the manufacturing device includes a molding assembly 100 including a mold table 110 and a male mold 120. The mold table 110 defines a depression 111 in its upper surface. The male mold 120 is adapted to be placed on a bottom surface of the depression 111 and has a height smaller than a depth of the depression 111. In this embodiment, the male mold 120 is a master mold for making other molds, by which final products mimicking the shape of the master mold can be fabricated. The mold is used, for example, to fabricate microneedles. In this case, the male mold 120 may include a substrate 121 and needles 122 projecting upright from an upper surface of the substrate 121. The needles are pointed protrusions. The male mold 120 may be made of a metal, silicon, a polymer with sufficient strength, or the like. The depth of the depression 111 refers to its dimension measured in a vertical direction. The height of the male mold 120 refers to the sum of dimensions of the substrate 121 and the needles 122 both measured in the vertical direction. The manufacturing device can be used to fabricate molds by transfer molding with higher production efficiency. Preferably, the bottom surface of the depression 111 has an area greater than that of the substrate 121. This can facilitate subsequent demolding.

Additionally, the upper surface of the substrate 121 is flat, and the manufacturing device further includes an adjustment mechanism 200 for driving the male mold 120 to move and making the upper surface of the substrate 121 horizontal. In a process of manufacturing a mold using the manufacturing device, it may be first determined whether the upper surface of the substrate 121 of the male mold 120 is horizontal. If no, the adjustment mechanism 200 is used to drive the male mold 120 to move until the upper surface of the substrate 121 becomes horizontal. A liquid raw material may be cast into the depression 111 so that the male mold 120 is submerged in the liquid raw material. Those skilled in the art will recognize that a mold can be obtained once the liquid raw material solidifies. A portion of the liquid raw material that has solidified between an upper surface thereof and the upper surface of the substrate 121 forms a substrate of the resulting mold.

Therefore, according to embodiments of the present invention, mold manufacturing begins only after horizontality of the upper surface of the substrate 121 is attained. Since the upper surface of the liquid raw material is per se horizontal, a uniform substrate thickness of the resulting mold can be obtained by ensuring parallelism of the upper surfaces of the substrate 121 and the liquid raw material. The mold obtained in this way can be used subsequently to fabricate microneedles with high yield. In the illustrated embodiment, flatness of the upper surface of the substrate 121 is preferred to be 0.05 mm or less, which can reduce errors and increase uniformity of the substrate. Those skilled in the art would appreciate that the adjustment mechanism 200 is adapted to make the upper surface of the substrate 121 horizontal through rocking the male mold 120 in a vertical plane. Optionally, both a lower surface of the substrate 121 and the bottom surface of the depression 111 may be flat. In this way, the upper surface of the substrate 121 will be located in a horizontal plane once the mold table 110 is moved by the adjustment mechanism 200 so that the bottom surface of the depression 111 is located in another horizontal plane. Those skilled in the art would appreciate that the bottom surface of the depression 111 and the upper surface of the substrate 121 are located in the different horizontal planes as a result of the adjustment due to a thickness of the substrate 121.

During the movement of the male mold 120 driven by the adjustment mechanism 200, a level may be used to monitor horizontality of the upper surface of the substrate 121. The level is preferred to be electronic or inductive. In some embodiments, the level is mounted to the upper surface of the substrate 121 and configured to directly monitor its horizontality. In some other embodiment, the adjustment mechanism 200 includes a motion table optionally having a flat upper surface for supporting (directly or indirectly) the molding assembly 100 in such a manner that the upper surface of the substrate 121 is parallel to the upper surface of the motion table. The motion table may be configured to move under the action of an external force (e.g., exerted by a third drive device), thereby causing the mold table 110 and hence the substrate 121 to move. In this case, the level may be disposed on the upper surface of the motion table and monitor horizontality of the upper surface of the substrate 121 indirectly by monitoring horizontality of the upper surface of the motion table. The motion table may be made of metal or marble.

Preferably, the manufacturing device further includes a heater assembly for heating the molding assembly 100. Heat from the heater assembly can be transmitted through the molding assembly 100 to the liquid raw material to facilitate and accelerate solidification of the liquid raw material at a predetermined temperature, shortening the time required for formation of the mold. Moreover, the solidification is allowed to occur at a constant temperature with little variation. Such variation can adversely affect the quality of the resulting mold. Optionally, the heater assembly may include an electric heating plate 310 provided on the motion table, with the molding assembly 100 being in turn provided on the electric heating plate 310 (i.e., the motion table indirectly supports the molding assembly 100 through the electric heating plate 310).

Additionally, the manufacturing device may further include a box 410 and a vacuum generation element 420. The box 410 is adapted to house the molding assembly 100, the electric heating plate 310 and the motion table. That is, mold formation is conducted within the box 410. The box 410 may define an air outlet (not labeled), an air inlet (not shown) and a mold removal port (not labeled). The vacuum generation element 420 is, for example, a vacuum pump or a vacuum generator. The vacuum generation element 420 is in communication with the air outlet and act to create a vacuum in the box 410. A first valve (not shown) may be disposed at the air inlet. When the first valve is opened, ambient air is allowed to enter the box 410. A door assembly 411 is disposed at the mold removal port. The door assembly 411 may be closed to close the mold removal port. Once the door assembly 411 is opened, an operator or mechanical mechanism (e.g., the mold removal assembly 620 detailed below) can take the mold out of the box 410 through the mold removal port. Preferably, the door assembly 411 is electrically controlled.

The manufacturing device may further include a feeder assembly 500 for casting the liquid raw material into the depression 111 through a wall of the box 410. Before the liquid raw material is cast, both the first valve and the door assembly 411 may be closed, and a vacuum may have been created in the box 410 by the vacuum generation element 420. That is, the liquid raw material is cast in a vacuum. This can avoid incorporation of air into the liquid raw material and dispense with degassing before solidification of the liquid raw material, resulting in time savings.

In some embodiments, the liquid raw material may be a mixture of at least two components. In these cases, the feeder assembly 500 may include a plurality of reservoirs 510, a plurality of metering mechanisms 520 and a mixing mechanism 530. Each reservoir 500 may store one component of the liquid raw material, and the metering mechanisms 520 may be disposed in correspondence with the respective reservoirs 510 and used to measure amounts of the respective components. The mixing mechanism 530 may be used to homogeneously mix the components to form the liquid raw material and cast the liquid raw material into the depression 111. Those skilled in the art would appreciate that the mixing mechanism 530 may be implemented as any suitable mixing or blending mechanism known in the art, which has an outlet optionally provided thereat with a second valve (not shown) and a pipe (not shown) communicating with the mixing mechanism 530 through the second valve. The pipe may be passed through the wall of the box 410 and used to supply the liquid raw material into the depression 111. In this way, the feeder assembly 500 allows in-situ preparation of the liquid raw material immediately before its use, avoiding variation of its viscosity or other properties during storage over time before use, which may adversely affect quality of the resulting mold.

The manufacturing device may further include a demolding and removal mechanism 600 for separating the mold from the molding assembly 100, moving it away from the molding assembly 100 and passing it out of the box 410.

In detail, the motion table may define a first through hole. The electric heating plate 310 may define a second through hole, and the mold table 110 may define a third through hole. The third, second and first through holes may be aligned with one another. The demolding and removal mechanism 600 may include a demolding assembly 610 and a mold removal assembly 620. In a non-limiting embodiment, the demolding assembly 610 may include a first drive device 611 and a first telescopic rod 612. The first drive device 611 may be deployed either within or outside of the box 410. The first telescopic rod 612 may be vertically erected, and its lower end may be connected to an output end of the first drive device 611. The first telescopic rod 612 may be upward inserted successively through the first, second and third through holes, and its upper end may be attached to the lower surface of the substrate 121. The first telescopic rod 612 may be driven by the first drive device 611 to extend or retract. It would be appreciated that the demolding assembly 610 is configured to move together with the male mold 120. For example, the first drive device 611 in the demolding assembly 610 may be coupled to the motion table so as to be able to move in synchronization therewith, thereby avoiding the first telescopic rod 612 from interfering with movement of the male mold 120. The mold removal assembly 620 may be disposed outside of the box 410 and may include second drive device 621, a second telescopic rod 622 and a support member 623. The second telescopic rod 622 may be oriented horizontally and disposed in positional correspondence with the mold removal port of the box 410. One end of the second telescopic rod 622 may be connected to an output end of the second drive device 621, and its other end may be connected to the support member 623. The second telescopic rod 622 may be driven by the second drive device 621 to extend or retract.

After the liquid raw material solidifies, the first drive device 611 may extend the first telescopic rod 612 and thereby raise the male mold 120 and the mold formed thereon away from the mold table 110. The second drive device 621 may then extend the second telescopic rod 622 and thereby move the support member 523 through the mold removal port (at this time, the door assembly 411 is opened) to a position under the mold so that the mold is supported thereon. Subsequently, the first drive device 611 may retract the first telescopic rod 612 and thereby cause the male mold 120 to move downward. Since the mold has been supported on the support member 623, this separates the mold from the male mold 120, followed by return of the male mold 120 to its rest position. Finally, the second drive device 621 may retract the second telescopic rod 622, moving the support member 623 together with the mold supported thereon out of the box 410.

In this embodiment, the demolding and removal mechanism can accomplish demolding and mold removal without manual intervention. Therefore, the manufacturing device can be used to manufacture molds in batches, wherein the demolding and mold removal step can be carried out immediately after the liquid raw material solidifies, without waiting for cooling of the molds. This can shorten the time required for the overall production process.

Further, the connection between the first telescopic rod 612 and the male mold 120 can stabilize the male mold 120, increasing consistency between molds from different batches. Furthermore, in this embodiment, the connection between the first telescopic rod 612 and the male mold 120 is preferred to be a detachable connection established threadedly or by any other suitable means, which can facilitate replacement of the male mold 120. Embodiments of the present invention are not limited to any particular shape of the support member 623, and it may be arbitrarily designed as practically needed, as long as the function of supporting the mold can be performed.

Those skilled in the art would appreciate that, typically, the lower surface of the substrate 121 and the bottom surface of the depression 111 do not tightly fit against each other due to a minimal gap present between them. When the liquid raw material is highly viscous and lack of fluidity, after being cast into the depression 111, it will not flow into the space between the lower surface of the substrate 121 and the bottom surface of the depression 111. However, in case of the liquid raw material being of low viscosity and high fluidity, it may flow between the lower surface of the substrate 121 and the depression 111 and even into the third, second and first through holes. Accordingly, in order to prevent flow of the liquid raw material between the lower surface of the substrate 121 and the bottom surface of the depression 111 and hence into the third, second and first through holes, a sealing ring may be added to the lower surface of the substrate 121 and/or the bottom surface of the depression 111.

The manufacturing device may further include a control assembly 700 for controlling various parameters of the molding process. For example, the control assembly 700 may be communicatively connected to the feeder assembly 500 and adapted to control the amounts and mixing speed of the components of the liquid raw material, as well as the feeding amount and speed of the liquid raw material. The heater assembly may further include a temperature sensor disposed in the electric heating plate 310, and the control assembly 700 may be communicatively connected to the heater assembly and adapted to control a temperature of the electric heating plate 310 within a predetermined range and thereby control a temperature of the molding assembly 100 within a predetermined range. The control assembly 700 may also be communicatively connected to the door assembly 411, the demolding and removal mechanism 600, the vacuum generation element 420 and the first valve and adapted to control opening and closing of the door assembly 411, demolding parameters of the demolding and removal mechanism 600, a vacuum of the box 410 and opening and closing of the first valve. In this way, the molding process can be precisely controlled, resulting in higher quality consistency among the molds manufactured in different batches. In this embodiment, the control assembly may be a one-piece structure, or may include a plurality of separate control elements, such as a first control element 710 and a second control element 720. The first control element 710 may be communicatively connected to the feeder assembly 500, the door assembly 411, the demolding and removal mechanism 600 and the first valve, and the second control element 720 may be communicatively connected to the heater assembly.

In embodiments of the present invention, there is also provided a manufacturing method for molds using the manufacturing device as defined above. The manufacturing method includes the steps as follows.

Step S10: Casting the liquid raw material into the depression 111 so that the male mold 120 is submerged in the liquid raw material. In this embodiment, the liquid raw material is cast into the depression 111 using the feeder assembly 500.

Step S20: Solidifying the liquid raw material to form a mold. In this embodiment, the liquid raw material preferably solidifies at a first predetermined temperature by heating the molding assembly 100 to the first predetermined temperature by the heater assembly 300.

Preferably, the manufacturing method further includes Step S00: driving the male mold 120 to move to make the upper surface of the male mold 120 horizontal.

Preferably, the molding method further includes Step S01: preheating the molding assembly 100. That is, after step S10 and before Step S20, the molding assembly 100 may be heated to a second predetermined temperature, accomplishing preheating of the molding assembly 100. The second predetermined temperature may be lower than or equal to the first predetermined temperature.

In case of the manufacturing device including the box 410 and the vacuum generation element 420, the molding method may include Step S02: activating the vacuum generation element 420 to create a vacuum in the box 410. Step S02 may be performed prior to Step S10, and before, during, or after Step S01.

The molding method may further include Step S11: opening the valve at the air inlet to allow air into the box 410 and thereby restore the pressure in the box 410 to the ambient atmospheric pressure. Step S11 may be carried out after or during Step S20.

The molding method may further include Step S30: transferring the mold out of the box using the demolding and removal mechanism. Step S30 may be performed after Step S20. Those skilled in the art would appreciate that, during continuous manufacturing, solidification of each mold may be immediately followed by its demolding and transfer by the demolding and removal mechanism 600. At this time, the molding assembly 100 has not been cooled down, and formation of the next mold can be directly conducted without preheating the molding assembly 100. That is, Step S01 can be carried out only once before the manufacturing of the first mold.

Embodiments of the present invention can be used to manufacture molds in batches with higher production efficiency. In particular, when the manufacturing device incorporates the adjustment mechanism, before manufacturing, the adjustment mechanism can be used to drive the male mold to move to make the upper surface of the substrate in the male mold horizontal. This enables the resulting molds to have a substrate with a uniform thickness, making the invention particularly suitable for the fabrication of microneedle molds. Further, in the present invention, a control mechanism may be used to maintain manufacturing parameters constant, enhancing quality consistency among the manufactured molds in different batches.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A manufacturing device for molds, comprising a molding assembly comprising a mold table and a male mold, the mold table having an upper surface defining a depression thereon, the male mold configured to be disposed on a bottom surface of the depression, the male mold having a height smaller than a depth of the depression.

2. The manufacturing device for molds according to claim 1, wherein the male mold comprises a substrate having a flat upper surface, wherein the manufacturing device further comprises an adjustment mechanism for driving the male mold to move and making the upper surface of the substrate horizontal.

3. The manufacturing device for molds according to claim 2, wherein both a lower surface of the substrate and the bottom surface of the depression are flat, wherein the adjustment mechanism is configured to drive the mold table to move so that the bottom surface of the depression is located in a horizontal plane, and accordingly drive the male mold to move so that the upper surface of the substrate is located in another horizontal plane.

4. The manufacturing device for molds according to claim 3, wherein the adjustment mechanism comprises a motion table configured to move under an action of an external force, the motion table disposed under the molding assembly so as to support the molding assembly thereon.

5. The manufacturing device for molds according to claim 3, wherein the depression is configured for loading a liquid raw material therein, wherein a sealing ring for preventing flow of the liquid raw material into a space between the lower surface of the substrate and the bottom surface of the depression is disposed on the lower surface of the substrate and/or the bottom surface of the depression.

6. The manufacturing device for molds according to claim 1, wherein the depression is configured for loading a liquid raw material therein so that the male mold is submerged in the liquid raw material which is to be solidified to form a mold, and wherein the manufacturing device further comprises a demolding and removal mechanism for separating the formed mold from the molding assembly.

7. The manufacturing device for molds according to claim 6, wherein the demolding and removal mechanism comprises a demolding assembly and a mold removal assembly, the demolding assembly comprising a first telescopic rod and a first drive device, the first telescopic rod disposed vertically, the first telescopic rod passed through the mold table and connected to the male mold, the first drive device configured to drive the first telescopic rod to extend or retract, the mold removal assembly comprising a second telescopic rod, a second drive device and a support member, the second telescopic rod disposed horizontally, the second drive device configured to drive the second telescopic rod to extend or retract, the support member connected to the second telescopic rod and configured to support the mold,
wherein the manufacturing device is configured so that, when the first drive device drives the first telescopic rod to extend, the first telescopic rod propels the male mold to move upward to separate the male mold and the formed mold thereon from the mold table, that when the second drive device drives the second telescopic rod to extend, the second telescopic rod propels the support member to move so that the formed mold is supported on the support member, and that when the first drive device drives the first telescopic rod to retract, the male mold is moved downward to separate the male mold from the formed mold.

8. The manufacturing device for molds according to claim 7, further configured so that, when the second drive device drives the second telescopic rod to retract, the second telescopic rod propels the formed mold to move away from the molding assembly.

9. The manufacturing device for molds according to claim 7, wherein the male mold is detachably connected to the first telescopic rod.

10. The manufacturing device for molds according to claim 7, further comprising a box and a vacuum generation element, the box defining thereon a mold removal port provided thereat with a door assembly, the door assembly configured to close the mold removal port, the vacuum generation element configured to create a vacuum in the box,
wherein both the molding assembly and the demolding assembly are at least partially disposed in the box; the mold removal assembly is disposed outside the box; and the second telescopic rod is configured to extend through the mold removal port so that the support member enters into the box to allow the formed mold to be supported thereon.

11. The manufacturing device for molds according to claim 1 or 10, further comprising a heater assembly for heating the molding assembly.

12. The manufacturing device for molds according to claim 11, further comprising a feeder assembly for casting a liquid raw material into the depression.

13. The manufacturing device for molds according to claim 12, wherein the liquid raw material comprises at least two components, wherein the feeder assembly comprises a plurality of reservoirs and plurality of metering mechanisms, each of the reservoirs configured to store one of the components of the liquid raw material, the metering mechanisms disposed in correspondence with the respective reservoirs and configured to measure amounts of the respective components of the liquid raw material; and
the feeder assembly further comprises a mixing mechanism for homogenously mixing the components of the liquid raw material and casting the mixed liquid raw material into the depression.

14. The manufacturing device for molds according to claim 13, further comprising a control assembly communicatively connected to the feeder assembly and configured to control at least one of the amounts of the components of the liquid raw material, a mixing speed of the components and a casting speed of the liquid raw material, and/or when the manufacturing device comprises the box and the vacuum generation element, the control assembly is further connected to the vacuum generation element and configured to control a degree of vacuum in the box, and/or the control assembly is further communicatively connected to the heater assembly and configured to control a temperature of the molding assembly within a predetermined range.

15. The manufacturing device for molds according to claim 1, wherein the male mold comprises a substrate and needles arranged on an upper surface of the substrate.

16. A manufacturing method for molds, based on the manufacturing device for molds according to any of claims 1 to 15, the manufacturing method comprising:
Step S10: casting a liquid raw material into the depression so that the male mold is submerged in the liquid raw material; and
Step S20: solidifying the liquid raw material to form a mold.

17. The manufacturing method for molds according to claim 16, further comprising, prior to Step S10, Step S00: driving the male mold to move so that an upper surface of a substrate in the male mold is located in a horizontal plane.

18. The manufacturing method for molds according to claim 16, wherein the liquid raw material comprises at least two components, and the manufacturing device further comprising the feeder assembly,
wherein Step S10 comprises the feeder assembly mixing the components at a mixture ratio of the liquid raw material and casting the mixed liquid raw material into the depression at a predetermined speed.

19. The manufacturing method for molds according to claim 16, wherein Step S20 comprises heating the molding assembly to a first predetermined temperature to solidify the liquid raw material.

20. The manufacturing method for molds according to claim 17, further comprising Step S01: heating the molding assembly to a second predetermined temperature,
wherein Step S01 is performed after Step S00 and before Step S10.

21. The manufacturing method for molds according to claim 20, wherein the molding assembly is housed in a box,
wherein the method further comprises Step S02: creating a vacuum in the box; Step S02 is performed before Step S10;
the method further comprises Step S 11: introducing air into the box to restore a pressure in the box to an ambient atmospheric pressure; and Step S11 is performed after Step S10.
